# EUROPÄISCHE PATENTSCHRIFT

(11) **EP 0 858 465 B1**
(45) Veröffentlichungstag und Bekanntmachung des Hinweises auf die Patenterteilung: **29.01.2003**
(21) Anmeldenummer: 96945860.3
(22) Anmeldetag: 31.10.1996
(51) Int. Cl.: C07K 14/435

(54) **PROTEIN MIT ANTI-TUMORWIRKUNG**
ANTI-TUMORAL PROTEIN
PROTEINE A ACTION ANTITUMORALE

(30) Priorität: 02.11.1995 DE 19540902
(43) Veröffentlichungstag der Anmeldung: 19.08.1998
(73) Patentinhaber: Bioxen Ltd., St. Helier, Jersey JE24UA (GB)
(72) Erfinder: PETZELT, Christian, D-10625 Berlin (DE)
(74) Vertreter: Schüssler, Andrea, Dr.
(86) Internationale Anmeldenummer: DE9602104
(87) Internationale Veröffentlichungsnummer: WO97016457

(56) Entgegenhaltungen:
- COMP. BIOCHEM. PHYSIOL., Bd. 105C, Nr. 2, 1993, Seiten 141-146, XP002034987 YAMAZAKI M.: "Antitumor and antimicrobial glycoproteins from sea hares."
- FEBS LETTERS, Bd. 377, 27.Dezember 1995, Seiten 373-376, XP002035003 TAKAMATSU N. ET AL.: "Molecular cloning of the defense factor in the albumen gland of the sea hare Aplysia kurodai."

## Beschreibung

Die Erfindung betrifft ein Protein mit Anti-Tumorwirkung, eine ein solches Protein kodierende DNA und ein Verfahren zur Hersteilung eines solchen Proteins sowie dessen Verwendung.

Bei Tumorerkrankungen werden häufig Chemotherapeutika eingesetzt. Diese wirken jedoch nicht selektiv, d.h. sie greifen nicht nur den zu behandelnden Tumor sondern auch gesundes Gewebe an. Dieses wird dadurch erheblich geschädigt. Es ist somit wünschenswert, Antitumor-Mittel zur Verfügung zu haben, mit denen Tumoren selektiv behandelt werden können.

Yamazaki et al., Comp. Biochem. Physiol., Vol. 105C, No. 2, pp. 141-146, 1993 beschreibt die Isolierung und Reinigung von Glykoproteinen aus der Meeresschnecke Aplysia kurodai. In dieser Veröffentlichung sind allerdings keinerlei Sequenzdaten enthalten.

Der vorliegenden Erfindung liegt daher die Aufgabe zugrunde, ein solches Mittel bereitzustellen.

Erfindungsgemäß wird dies durch die Gegenstände in den Patentansprüchen erreicht.

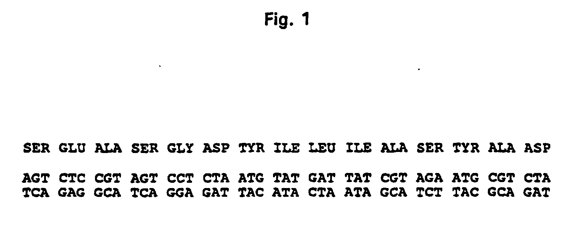

Gegenstand der Erfindung ist somit ein Protein mit Anti-Tumorwirkung, wobei das Protein die Aminosäuresequenz von Fig. 1 umfaßt.

Die vorliegende Erfindung beruht auf der Erkenntnis des Anmelders, daß in Meeresschnecken, insbesondere in Aplysia punctata, ein Protein vorliegt, das Tumorzellen, nicht aber gesunde Zellen abtötet. Dieses Protein umfaßt die in Fig. 1 dargestellte Aminosäuresequenz.

Ein erfindungsgemäßes Protein kann durch übliche Verfahren bereitgestellt werden. Günstig ist es, die Meeresschnecke A. punctata leicht zu drücken, wodurch sie ein blaudunkelviolettes Sekret abgibt. Dieses Sekret wird mit PBS verdünnt und einer differentiellen Zentrifugation unterzogen. Danach wird es mit Ammoniumsulfat fraktioniert ausgesalzen und einer Ionenaustauschchromatographie an einer DE-52 Säule sowie einer Polyacrylamid-Gelektrophorese unterworfen. Die aufgetrennten Proteine werden teilweise auf eine PVDF-Membran übertragen und mit Sulforhodamin B angefärbt. Entsprechend der sichtbaren Banden werden die Proteine aus dem Gel herausgeschnitten und auf ihre Anti-Tumorwirkung gegenüberTumorzellen getestet. Ein erfindungsgemäßes Protein, das selektiv gegen Tumorzellen wirkt, wird identifiziert.

Ein weiterer Gegenstand der Erfindung ist eine für ein vorstehendes Protein kodierende DNA. Diese kann z.B. eine genomische DNA oder eine cDNA sein. Bevorzugt ist eine DNA, die folgendes umfaßt:
(a) die DNA von Fig. 1, oder
(b) eine mit der DNA von (a) über den degenerierten genetischen Code verwandte DNA.

Zur Herstellung einer erfindungsgemäßen DNA ist es günstig, von einer cDNA-Expressionsbibliothek auszugehen, die von mRNA einer Meeresschnecke, insbesondere von A. punctata, erstellt ist. Eine solche Bibliothek kann mit polyklonalen bzw. monoklonalen Antikörpern, die gegen ein erfindungsgemäßes Protein gerichtet sind, gescreent werden. Das Herstellen solcher Antikörper erfolgt durch übliche Verfahren. Durch das Screenen werden positive Klone identifiziert. Diese können subkloniert und sequenziert werden, wodurch eine erfindungsgemäße DNA identifiziert wird.

Eine erfindungsgemäße DNA kann in einem Vektor bzw. Expressionsvektor vorliegen. Beispiele solcher sind dem Fachmann bekannt. Im Falle eines Expressionsvektors für E.coli sind dies z.B. λgt11, pGEMEX, pUC-Derivate, pGEX-2T, pET3b und PQE-8. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A.

Der Fachmann kennt geeignete Zellen, um eine erfindungsgemäße, in einem Expressionsvektor vorliegende DNA zu exprimieren. Beispiele solcher Zellen umfassen die E.coli-Stämme HB101, DH1, x1776, JM101, JM 109, BL21 und SG13009, den Hefe-Stamm Saccharomyces cerevisiae und die tierischen Zellen L, 3T3, FM3A, CHO, COS, Vero und HeLa sowie die Insektenzellen Sf9.

Der Fachmann weiß, in welcher Weise eine erfindungsgemäße DNA in einen Expressionsvektor inseriert werden muß. Ihm ist auch bekannt, daß diese DNA in Verbindung mit einer für ein anderes Protein bzw. Peptid kodierenden DNA inseriert werden kann, so daß die erfindungsgemäße DNA in Form eines Fusionsproteins exprimiert werden kann.

Des weiteren kennt der Fachmann Bedingungen, transformierte bzw. transfizierte Zellen zu kultivieren. Auch sind ihm Verfahren bekannt, das exprimierte Protein zu isolieren und zu reinigen. Ein vorstehendes, rekombinant hergestelltes Protein, das auch ein Fusionsprotein sein kann, ist somit ebenfalls Gegenstand der Erfindung.

Ein weiterer Gegenstand der vorliegenden Erfindung ist ein gegen ein vorstehendes Protein bzw. Fusionsprotein gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

Erfindungsgemäße Proteine zeichnen sich dadurch aus, daß sie Tumoren selektiv abtöten. Gesunde Zellen werden durch sie nicht beeinträchtigt. Dies verleiht der vorliegenden Erfindung eine außerordentliche Bedeutung für die Behandlung von Tumorerkrankungen. Ferner weisen Daten darauf hin, daß erfindungsgemäße Proteine auch antivirale Wirkung haben. Ihr Einsatz zur Behandlung von Virus-Erkrankungen ist somit geboten. Desweiteren können erfindungsgemäße Proteine für diagnostische Zwecke aller Art eingesetzt werden.

Weiterhin eignen sich erfindungsgemäße Antikörper, den Wirkmechanismus vorstehender Proteine zu untersuchen, wodurch es möglich ist, die Selektivität und Aktivität dieser Proteine noch zu steigern.

Darüberhinaus können diese Nukleinsäuren für therapeutische Maßnahmen verwendet werden. Beispielsweise können die Nukleinsäuren in übliche Expressionsvektoren inseriert werden und diese in Personen mit einem Tumor eingeschleust werden. Günstigerweise erfolgt die Einschleusung dabei direkt in den Tumor. Durch Expression der Nukleinsäuren werden im Körper und/oder im Tumor die Proteine exprimiert, die dann den Tumor selektiv abtöten.

### Kurze Beschreibung der Zeichnungen:

- Fig. 1: zeigt die von einem erfindungsgemäßen Protein umfaßte Aminosäuresequenz und die daraus abgeleitete Basensequenz.

Die folgenden Beispiele erläutern die Erfindung.

### Beispiel 1: Isolierung eines erfindungsgemäßen Proteins

Die Meeresschnecke *Aplysia punctata* wurde leicht gedrückt, wodurch sie ein blaudunkelviolettes Sekret abgab. Dieses Sekret wurde 1:10 mit PBS verdünnt. Das verdünnte Sekret wurde einer differentiellen Zentrifugation, einem fraktionierten Aussalzen mit Ammoniumsulfat in einem üblichen Puffer und einer lonenaustauschchromatographiean einer DE-52 Säule (Elutionsmittel: Puffer, pH 7,2, kontinuierlicher NaCI-Gradient von 10 mM bis 1 mM NaCl) unterzogen. Anschließend erfolgte eine Polyacrylamid-Gelelektrophorese unter Verwendung eines 10 %igen Polyacrylamidgels. Die aufgetrennten Proteine wurden teilweise auf eine PVDF-Membran übertragen und mit Sulforhodamin B angefärbt. Entsprechend der sichtbaren Banden wurden aus dem Gel die einzelnen Proteine herausgeschnitten und auf ihre Anti-Tumorwirkung getestet. Hierzu wurden sie mit Tumorzellen inkubiert und es wurde auf das Auftreten von Abtötungserscheinungen, wie Kontraktion der Zellen, gewartet.

Ein erfindungsgemäßes Protein wurde identifiziert. Eine Sequenzierung zeigte, daß dieses Protein die Aminosäuresequenz von Fig. 1 umfaßt.

### Beispiel 2: Wirkung eines erfindungsgemäßen Proteins auf Tumorzellen und gesunde Zellen

Das in Beispiel 1 beschriebene Protein wurde mit folgenden Zellinien inkubiert:
T47-Zellen (menschlicher Brustkrebs)
HeLa-Zellen (menschliches Uteruskarzinom)
A-204-Zellen (menschliches Rhabdomyosarcom)

Diese Zellinien zeigten nach ca. 3 h die ersten Anzeichen von Abtötung, d.h. Kontraktion und Verlust der Oberflächenadhäsion wurden erkennbar. Nach 4 - 5 h waren die Zellen abgetötet.

Im Gegensatz dazu wurden gesunde Zellen nicht durch ein erfindungsgemäßes Protein beeinflußt bzw. abgetötet. Beispielsweise wurde die Beweglichkeit und das Befruchtungspotential von Spermien sowie das Befruchtungspotential von Eizellen nicht beeinflußt. Desweiteren zeigten sich keine Auswirkungen auf bereits befruchtete Eizellen, z.B. Einzell-Embryonen, wenn das erfindungsgemäße Protein in diese mikroinjiziert wurde. Ferner wurden keine nachteiligen Auswirkungen beobachtet, wenn das erfindungsgemäße Protein in folgende Lebewesen direkt injiziert wurde: Seeanemone, Seestern, Steinbutt und Maus. Weiterhin wurden keine nachteiligen Effekte beobachtet, wenn das erfindungsgemäße Protein direkt mit offenen Wunden von Menschen in Kontakt gebracht wurde.

Vorstehende Versuche zeigen, daß ein erfindungsgemäßes Protein selektiv Tumorzellen abtötet, gesundes Gewebe aber nicht beeinträchtigt.

## Patentansprüche

1. Protein mit Anti-Tumorwirkung umfassend die Aminosäuresequenz von Fig. 1.

2. DNA, kodierend für das Protein nach Anspruch 1, wobei die DNA umfaßt:
(a) die DNA von Fig. 1, oder
(b) eine mit der DNA von (a) über den degenerierten genetischen Code verwandte DNA.

3. Expressionsplasmid, umfassend die DNA nach Anspruch 2.

4. Transformante, enthaltend das Expressionsplasmid nach Anspruch 3.

5. Verfahren Zur Herstellung des Proteins nach Anspruch 1, umfassend die Kultivierung der Transformante nach Anspruch 4 unter geeigneten Badingungen.

6. Antikörper, spezifisch gerichtet gegen das Protein nach Anspruch 1.

7. Verwendung des Proteins nach Anspruch 1 zur Herstellung eines pharmazeutischen Zusammensetzung zur Behandlung von Tumoren.

8. Verwendung der DNA nach Anspruch 2 zur Herstellung eines pharmazeutischen Zusammensetzung zur Behandlung von Tumoren.

## Claims

1. A protein having an anti-tumoral effect, comprising the amino acid sequence of fig. 1.

2. A DNA coding for the protein according to claim 1, wherein the DNA comprises:
(a) the DNA of fig. 1 or
(b) a DNA related to the DNA of (a) via the degenerated genetic code.

3. An expression plasmid, comprising the DNA according to claim 2.

4. A transformant containing the expression plasmid according to claim 3.

5. A method of preparing the protein according to claim 1, comprising culturing the transformant according to claim 4 under suitable conditions.

6. Antibodies directed specifically against the protein according to claim 1.

7. Use of the protein according to claim 1 for the preparation of a pharmaceutical composition for treating tumors.

8. Use of the DNA according to claim 2 for the preparation of a pharmaceutical composition for treating tumors.

## Revendications

1. Protéine à action anti-tumorale, comprenant la séquence d'aminoacides de la fig. 1.

2. ADN, codant la protéine suivant la revendication 1, qui comprend :
(a) l'ADN de la fig. 1, ou
(b) un ADN apparenté à l'ADN de (a) par le code génétique dégénéré.

3. Plasmide d'expression, comprenant l'ADN suivant la revendication 2.

4. Transformants, contenant le plasmide d'expression suivant la revendication 3.

5. Procédé de production de la protéine suivant la revendication 1, comprenant la culture des transformants suivant la revendication 4 dans des conditions appropriées.

6. Anticorps dirigés spécifiquement contre la protéine suivant la revendication 1.

7. Utilisation de la protéine suivant la revendication 1 pour la préparation d'une composition pharmaceutique destinée au traitement de tumeurs.

8. Utilisation de l'ADN suivant la revendication 2 pour la préparation d'une composition pharmaceutique destinée au traitement de tumeurs.
